# EUROPEAN PATENT APPLICATION

(11) **EP 4 574 998 A1**
(43) Date of publication of application: **25.06.2025**
(21) Application number: 23307321.2
(22) Date of filing: 21.12.2023
(51) Int. Cl.: C12Q 1/6886

(54) **METHYLATED BIOMARKERS FOR USE IN LUNG CANCER DIAGNOSIS**

(71) Applicant: Université de Franche-Comté, 25030 Besançon Cedex (FR); Institut National de la Santé et de la Recherche Médicale, 75013 Paris (FR); Etablissement français du sang, 93218 La Plaine Saint Denis Cedex (FR); Centre Hospitalier Universitaire De Dijon, 21079 Dijon Cedex (FR)
(72) Inventor: SELMANI, Zohair, 25000 Besançon (FR); OVERS, Alexis, 25000 Besançon (FR); TOURNIER, Benjamin, 21850 Saint Apollinaire (FR); PEIXOTO, Paul, 25870 Cussey sur l'Ognon (FR); HERVOUET, Eric, 25360 Bouclans (FR)
(74) Representative: Gevers & Orès

(57) **Abstract**

The present invention relates to the detection of at least one methylated biomarker derived from lung cancer cells genomic DNA in biological samples and methods of diagnosing or monitoring lung cancer using such methylated biomarker(s).

## Description

### FIELD OF THE INVENTION:

The present invention relates to the detection of methylated biomarker(s) derived from lung cancer cells genomic DNA in biological samples and methods of diagnosing or monitoring lung cancer using such methylated biomarker(s).

### BACKGROUND OF THE INVENTION:

Lung cancer is one of the most commonly diagnosed form of cancer (11.6% of the total cases) and the leading cause of global cancer mortalities (International Agency for Research on Cancer, Global cancer statistics 2018).

Lung tumors are divided into two broad categories by the World Health Organization (WHO); non-small cell lung cancer (NSCLC), comprising 80-85% of all lung cancer cases, and small cell lung cancer (SCLC), constituting the other 15% incidences (Travis WD et al. The 2015 WHO classification of lung tumors: impact of genetic, clinical and radiologic advances since the 2004 classification. J Thor Oncol Publication Int Assoc Study Lung Cancer. 2015;10(9):1243-1260).

NSCLC are essentially adenocarcinomas (70%) and squamous cell cancer (20%). NSCLC are classified in various stages according to the TNM Classification of Malignant Tumours, 8th edition of Union of International Cancer Control.

NSCLC prognosis depends on the stage of the disease with a 5-year survival from 50 % for local stages to less than 5% for metastatic stages (Malvezzi, M, Ann Oncol. May 1;28(5):1117-1123, 2017). Therefore, early diagnosis of NSCLC is a major public health issue.

The National Lung Screening Trial (NLST) demonstrated a 20% reduction in lung cancer mortality using low-dose computed tomography (CT) screening. However, this survival benefit comes at the price of detecting many indeterminate pulmonary nodules with an overall false positive rate of 96.4%. (National Lung Screening Trial Research Team, Aberle DR, Adams AM, Berg CD, Black WC, Clapp JD, et al. Reduced lung-cancer mortality with low-dose computed tomographic screening. N Engl J Med. 2011;365:395-409). This has led to a cautious adoption of CT screening, because complications, and even deaths and to the search for specific biomarkers to improve the specificity of CT screening. One trail for biomarker is the study of DNA methylation. However, nowadays none biomarker based on DNA methylation has shown sufficient sensitivity and/or specificity for lung cancer screening (Palmisano WA et al. Predicting lung cancer by detecting aberrant promoter methylation in sputum. Cancer Res. 2000;60:5954-8, Brock MV et al. DNA methylation markers and early recurrence in stage I lung cancer. New England Journal of Medicine. 2008;358:1118-28, Ostrow KL, Hoque MO, Loyo M, Brait M, Greenberg A, Siegfried JM, et al. Molecular analysis of plasma DNA for the early detection of lung cancer by quantitative methylation-specific PCR. Clin Cancer Res. 2010;16:3463-72. Sandoval J, et al. A prognostic DNA methylation signature for stage I non-small-cell lung cancer. J Clin Oncol. 2013;31:4140-7, Nawaz I, Qiu X, Wu H, Li Y, Fan Y, Hu L-F, et al. Development of a multiplex methylation specific PCR suitable for (early) detection of non-small cell lung cancer. Epigenetics. 2014;9:1138-48, A. Hubert et al., Clin Cancer Res. 2017 Apr 15; 23(8): 1998-2005).

### SUMMARY OF THE INVENTION:

Now, the applicants have found epigenetic biomarkers specific of lung cancer, in particular of non-small cell lung cancer (NSCLC). These biomarkers were validated *in silica* and *in vivo* on liquid biopsy as shown in the Examples.

Therefore, in a first aspect of the invention, it is provided a method of diagnosing lung cancer, preferably a non-small cell lung cancer (NSCLC), in a subject, the method of diagnosing comprising the step of detecting DNA methylation at one or more CpG sites within at least one polynucleotide biomarker, in a biological sample obtained from said subject,
wherein said at least one polynucleotide biomarker is selected from the group consisting of:
   - a first polynucleotide biomarker having a sequence comprised in the nucleic acid sequence SEQ ID NO: 1,
   - a second polynucleotide biomarker having a sequence comprised in the nucleic acid sequence SEQ ID NO: 2, and
   - a third polynucleotide biomarker having a sequence comprised in the nucleic acid sequence SEQ ID NO: 3.
wherein the presence of detected DNA methylation at one or more CpG sites is indicative that the subject has a lung cancer.

In a second aspect of the invention, it is provided a method of monitoring a lung cancer, preferably a NSCLC, in a subject, the method of monitoring comprising the steps of :
(a) determining the level of DNA methylation at one or more CpG sites within at least one polynucleotide biomarker, in a first biological sample obtained from said subject at a first time point and
   wherein said at least one polynucleotide biomarker is selected from the group consisting of:
   - a first polynucleotide biomarker having a sequence comprised in the nucleic acid sequence SEQ ID NO: 1,
   - a second polynucleotide biomarker having a sequence comprised in the nucleic acid sequence SEQ ID NO: 2, and
   - a third polynucleotide biomarker having a sequence comprised in the nucleic acid sequence SEQ ID NO: 3,
(b) determining the level of DNA methylation at one or more CpG sites within said at least one polynucleotide biomarker in a second biological sample obtained from said subject later at a second time point, and
(c) comparing the level of DNA methylation determined in the second biological sample with the level of DNA methylation determined in the first biological sample,
   wherein an increase between the level of DNA methylation determined in the second biological sample and the level of DNA methylation determined in the first biological sample is indicative that the lung cancer is progressing,
   wherein a decrease between the level of DNA methylation determined in the second biological sample and the level of DNA methylation determined in the first biological sample is indicative that the lung cancer is not progressing.

In a third aspect of the invention, it is provided a method of monitoring the efficiency of a treatment in a subject suffering from a lung cancer, preferably a NSCLC, the method of monitoring comprising the steps of:
(a) determining the level of DNA methylation at one or more CpG sites within at least one polynucleotide biomarker, in a biological sample obtained from said subject before or at the beginning of the treatment,
   wherein said at least one polynucleotide biomarker is selected from the group consisting of:
   - a first polynucleotide biomarker having a sequence comprised in the sequence SEQ ID NO: 1,
   - a second polynucleotide biomarker having a sequence comprised in the sequence SEQ ID NO: 2, and
   - a third polynucleotide biomarker having a sequence comprised in the sequence SEQ ID NO: 3,
(b) determining the level of DNA methylation at one or more CpG sites in said at least one polynucleotide biomarker, in a biological sample obtained from said subject during or after the treatment, and
(c) comparing the level of DNA methylation determined before or at the beginning of the treatment with the level of DNA methylation determined during or after the treatment,
   - wherein an increase or an equality between the level of DNA methylation determined before or at the beginning of and during or after the treatment is indicative that the treatment was not efficient,
   - wherein a decrease between the level of DNA methylation determined before or at the beginning of and during or after the treatment is indicative that the treatment is efficient.

In a fourth aspect of the invention, it is provided a method for treating lung cancer in a subject, the method of treating comprising the steps of:
(a) detecting DNA methylation at one or more CpG sites within at least one polynucleotide biomarker, in a biological sample obtained from said subject,
   wherein said at least one polynucleotide biomarker is selected from the group consisting of:
   - a first polynucleotide biomarker having a sequence comprised in the nucleic acid sequence SEQ ID NO: 1,
   - a second polynucleotide biomarker having a sequence comprised in the nucleic acid sequence SEQ ID NO: 2, and
   - a third polynucleotide biomarker having a sequence comprised in the nucleic acid sequence SEQ ID NO: 3,
(b) treating the subject for the lung cancer, if the presence of DNA methylation is detected.

In another aspect of the invention, it is provided a composition of primers for detecting and/or determining the level of DNA methylation at one or more CpG sites within at least one polynucleotide biomarker, wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3.

In some embodiments, the composition of primers comprises at least one pair of primers selected from the group consisting of:
- a pair of primers able to produce the amplicon having the nucleic acid sequence selected from the group consisting of SEQ ID NO: 7, SEQ ID NO: 16 and SEQ ID NO: 19,
- a pair of primers able to produce the amplicon having the nucleic acid sequence selected from the group consisting of SEQ ID NO: 8, SEQ ID NO: 17 and SEQ ID NO: 20,and
- a pair of primers able to produce the amplicon having the nucleic acid sequence selected from the group consisting of SEQ ID NO: 9, SEQ ID NO: 18 and SEQ ID NO: 21.

In some embodiments, the composition of primers comprises at least one pair of primers selected from the group consisting of:
- a pair of primers comprising a primer having the nucleic acid sequence SEQ ID NO: 22 and a primer having the nucleic acid sequence SEQ ID NO: 23,
- a pair of primers comprising a primer having the nucleic acid sequence SEQ ID NO: 24 and a primer having the nucleic acid sequence SEQ ID NO: 25, and
- a pair of primers comprising a primer having the nucleic acid sequence SEQ ID NO: 26 and a primer having the nucleic acid sequence SEQ ID NO: 27.

In another aspect of the invention, it is provided a kit for detecting DNA methylation comprising:
- means for detecting and/or determining the level of DNA methylation at one or more CpG sites within at least one polynucleotide biomarker, wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3,
and optionally reagents for DNA amplification.

Another aspect of the invention is the use of the composition of primers of the invention, or of the kit of the invention for diagnosing and/or monitoring a lung cancer, preferably NSCLC in a subject.

In some embodiments of the various aspects of the invention:
- the DNA methylation is detected and/or its level determined within at least two of the polynucleotide biomarkers, preferably the three polynucleotide biomarkers ;
- the first polynucleotide biomarker has a sequence which is comprised in the nucleic acid sequence SEQ ID NO: 4, preferably a sequence which comprises or consists of the nucleic acid sequence SEQ ID NO: 7;
- the second polynucleotide biomarker has a sequence which is comprised in the nucleic acid sequence SEQ ID NO: 5, preferably a sequence which comprises or consists of the nucleic acid sequence SEQ ID NO: 8 ;
- the third polynucleotide biomarker has a sequence which is comprised in the nucleic acid sequence SEQ ID NO: 6, preferably a sequence which comprises or consists of the nucleic acid sequence SEQ ID NO: 9;
- the one or more CpG sites in the first polynucleotide biomarker is cg00995986; :
- the one or more CpG sites in the second polynucleotide biomarker is cg03224572 ;
- the one or more CpG sites in the second polynucleotide biomarker is cg01452847.

The locations of these CpG sites are given in the Illumina HumanMethylation450K Manifest.

### BRIEF DESCRIPTION OF THE DRAWINGS

**Figure 1** is a map of the target region related to *MEIS1.*
**Figure 2** represents the nucleic sequence of the target region related to *MEIS1* before (upper sequence, SEQ ID NO: 31) and after bisulfite treatment assuming that all the CpGs are methylated (lower sequence SEQ ID NO: 32). The bisulfite treatment converts the unmethylated cytosine into uracil while methylated cytosine are not converted. Between the two sequences, the modifications due to the bisulfite conversion are shown as follows:
   "|" corresponds to nucleotides which are not cytosines and are not modified,
   ":" corresponds to non-CpG cytosines which are necessarily converted,
   "++" corresponds to CpGs. In figure 2 (as well as figures 4 and 6), the cytosines are assumed to be methylated and thus not modified by bisulfite conversion.
   Amplicon and converted amplicon (assuming all CpGs are methylated) according to one preferred embodiment of the invention are in bold.
   Sequences where primers hybridize are underlined in full line. Sequence where the hydrolysis probe hybridizes is underlined in dotted line.

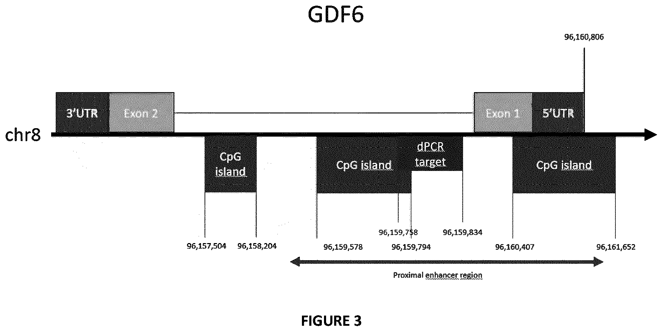

**Figure 3** is a map of the target region related to *GDF6.*

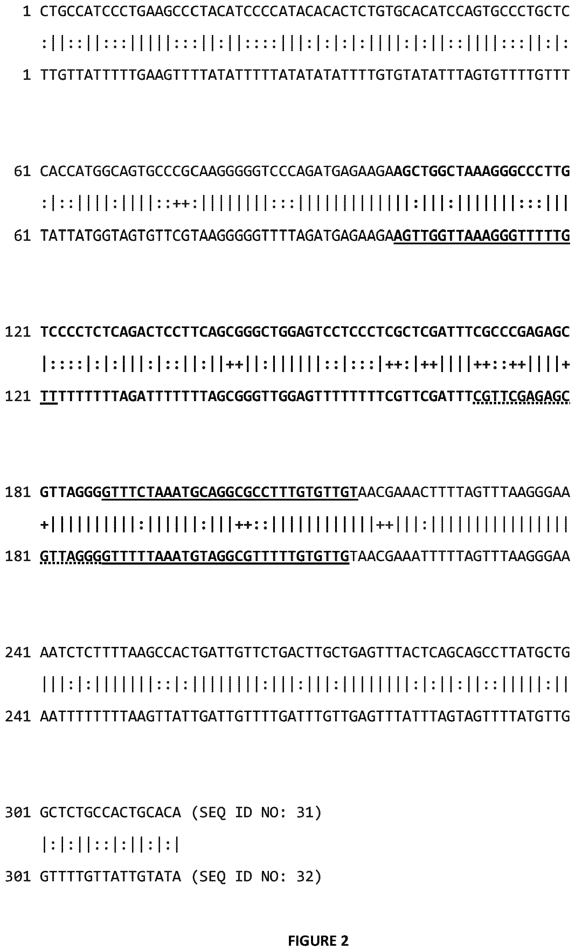

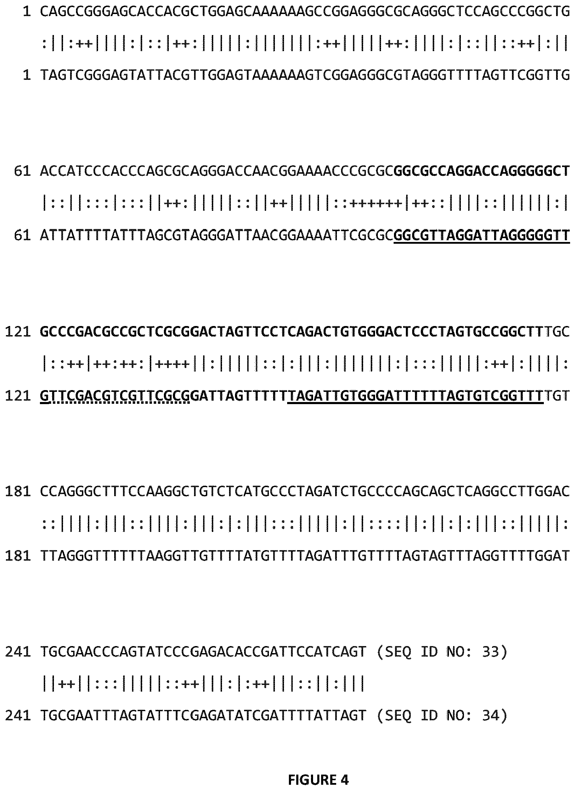

**Figure 4** represents the nucleic sequence of the target region related to *GDF6* before (upper sequence, SEQ ID NO: 33) and after bisulfite treatment assuming that all the CpGs are methylated (lower sequence SEQ ID NO: 34). The legend is the same as in figure 2.

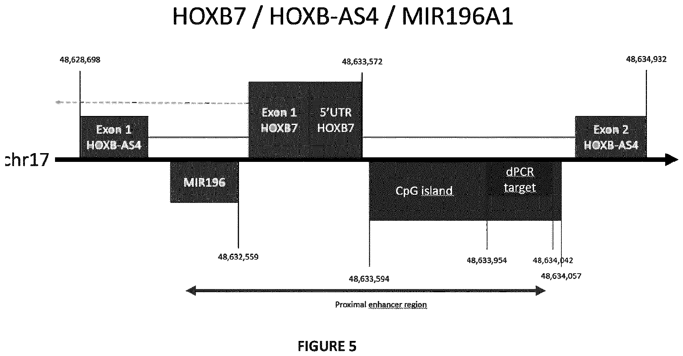

**Figure 5** is a map of the target region related to *MIR196A1.*

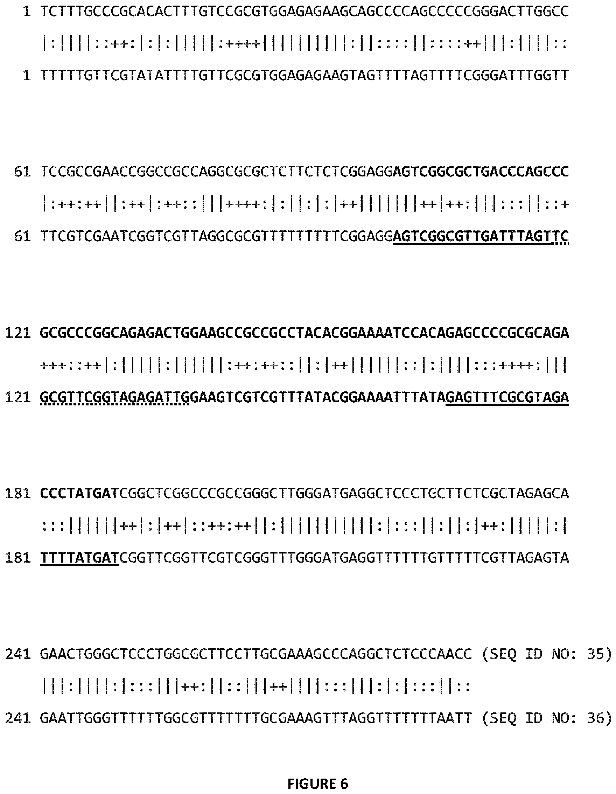

**Figure 6** represents the nucleic sequence of the target region related to *MIR196A1* before (upper sequence, SEQ ID NO: 35) and after bisulfite treatment assuming that all the CpGs are methylated (lower sequence SEQ ID NO: 36). The legend is the same as in figure 2.

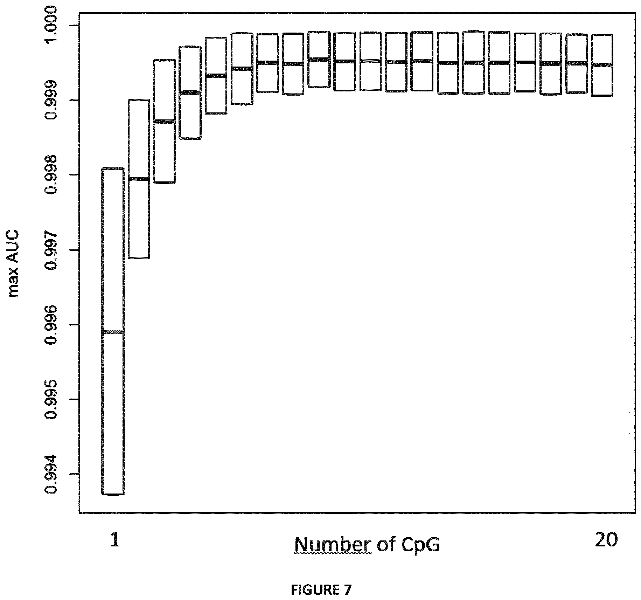

**Figure 7** is a plot showing the relation of the maximum areas under the curve (AUC) with IC95 (y-axis) to the numbers of CpG in the panel (x-axis). The AUC values represented by the horizontal bars and the confidence intervals by the boxes surrounding them.

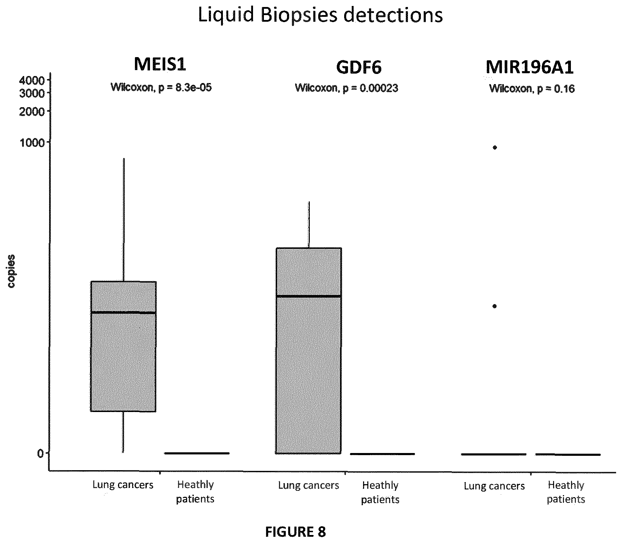

**Figure 8** shows box-and-whisker plots of number of methylated copies of genes in plasma of 15 subjects having lung adenocarcinoma (on left) versus plasma of 15 subjects with no tumor (on right) for each marker related to *MEIS1* gene, *GDF6* gene and *MIR196A1* gene.

### DETAILED DESCRIPTION OF THE INVENTION

### A. Method of detecting DNA methylation

One aspect of the invention relates to a method of detecting DNA methylation at one or more CpG sites within at least one polynucleotide biomarker,
wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3.

The term "detecting DNA methylation" as used herein refers to at least qualitatively analyzing for the presence or absence of methylated target DNA.

Advantageously, the at least one polynucleotide biomarker is from genomic DNA. Preferably, it is a sequence within the genomic DNA (region) that is limited in length (e.g. at least 30 to 1000, more preferably 50 to 300 and even more preferably 75 to 200 or 75 to 150 nucleotides long).

In a preferred embodiment, the genomic DNA is a cell-free DNA. The term "cell-free DNA" as used herein or its synonyms "cfDNA", and "extracellular DNA", "circulating DNA" and "free circulating DNA" refers to DNA that is not comprised within an intact cell in the respective body fluid which is the sample or from which the sample is derived, but which is free in the body liquid sample.

Methylation is preferably determined at 1 or more, 2 or more, 3 or more, 4 or more, or 5 or more, or 6 or more, or 7 or more (e.g. 7, 8, 9, 10 or more) CpG sites of the target DNA. Usually, the CpG sites analyzed are comethylated in cancer.

The present invention also relates to a method of determining the level of DNA methylation, the method comprising the step of determining the level of DNA methylation at one or more CpG sites within at least one polynucleotide biomarker:
wherein said at least one polynucleotide biomarker is selected from the group consisting of the first polynucleotide biomarker, the second polynucleotide biomarker and the third polynucleotide biomarker as defined above and hereinafter.

"Determining the level of DNA methylation" can mean determining a quantitative value (using any convenient metric) that represents the level of methylation at one or more CpG sites. The level of methylation can be expressed in arbitrary units associated with a particular assay (e.g., fluorescence units, e.g., mean fluorescence intensity (MFI)), or can be expressed as an absolute value with defined units (e.g., number of methylated CpG sites in a cfDNA gene, frequency of methylation at a CpG site in cfDNA, etc.). Additionally, the level of methylation at a CpG site can be compared to the methylation level of one or more additional CpG sites to derive a normalized value that represents a normalized methylation level. The level of DNA methylation in a sample may be the number of copies with a given DNA methylation at one or more CpG sites in said sample.

The terms "quantity", "amount", and "level" are used interchangeably herein and may refer to an absolute quantification of a molecule or an analyte in a sample, or to a relative quantification of a molecule or analyte in a sample, i.e., relative to another value such as relative to a reference value as taught herein, or to a range of values for the biomarker.

Therefore, the methods of the present invention may comprise the step of comparing the DNA methylation detected and/or level of DNA methylation determined to a reference value. The reference value or range value can be obtained from a single subject or from a group of subjects. For example, a reference value can be obtained from a single subject or a group subject not having a lung cancer, preferably not having a NSCLC. On contrary, the reference value can be obtained from a single subject or a group subject having a lung cancer, preferably having a NSCLC.

Typically, the DNA methylation is detected and/or the level of DNA methylation is determined in a biological sample obtained from a subject.

Advantageously, the methods of the invention are *in vitro.*

DNA methylation can be detected or its level can be determined by various means known in the art, e.g. autoradiography, silver staining or ethidium bromide staining, methylation sensitive single nucleotide extension (MS-SNUPE), methyl-binding proteins, antibodies for methylated DNA, methylation-sensitive restriction enzymes etc., preferably by sequencing, e.g. next-generation-sequencing (NGS), or by real-time PCR, e.g. multiplex real time PCR, or by digital PCR (dPCR).

In a real-time PCR, this is done by detecting a methylation-specific oligonucleotide probe during amplifying the converted (e.g. bisulfite converted) target DNA methylation specifically using methylation-specific primers or a methylation-specific blocker with methylation-specific primers or preferably methylation-unspecific primers.

Digital PCR (dPCR) is a quantitative PCR in which a PCR reaction mixture is partitioned into individual compartments (e.g. wells or water-in-oil emulsion droplets) resulting in either 1 or 0 targets being present in each compartment. Following PCR amplification, the number of positive vs negative reactions is determined and the quantification is by derived from this result statistically, preferably using Poisson statistics.

"Next-generation-sequencing" (NGS) refers to sequencing the bases of a small fragment of DNA which are sequentially identified from signals emitted as each fragment is re-synthesized from a DNA template strand.

The term "convert" means "convert, in DNA, cytosine unmethylated in the 5-position to uracil or another base that does not hybridize to guanine". It refers to a process of chemically treating the DNA in such a way that all or substantially all of the unmethylated cytosine bases are converted to uracil bases, or another base which is dissimilar to cytosine in terms of base pairing behavior, while the 5-methylcytosine bases remain unchanged. The conversion of unmethylated cytosine bases within the DNA sample is conducted with a converting agent.

The term "converting agent" as used herein relates to a reagent capable of converting an unmethylated cytosine to uracil or to another base that is detectably dissimilar to cytosine in terms of hybridization properties. The converting agent is preferably a bisulfite such as disulfite, or hydrogen sulfite.

### First biomarker

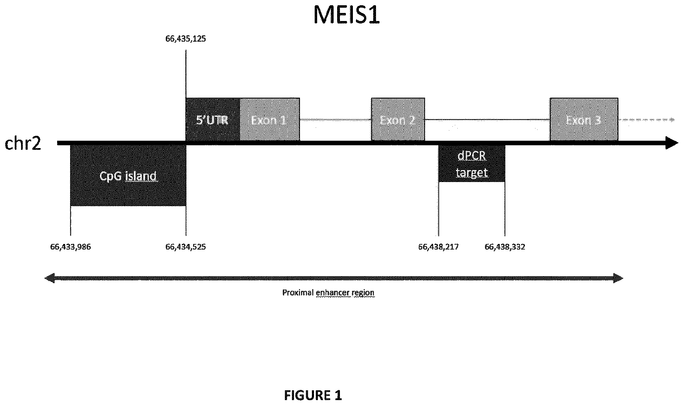

As shown in figure 1, the first polynucleotide biomarker is inside the gene *MEIS1,* typically between exon 2 and exon 3.

In some embodiments, the first polynucleotide biomarker has a sequence comprised in the nucleic acid sequence SEQ ID NO: 1.

In some embodiments, the first polynucleotide biomarker has a sequence which is comprised in the nucleic acid sequence SEQ ID NO: 4.

In some embodiments, the sequence of the first polynucleotide biomarker comprises the nucleic acid sequence SEQ ID NO: 7. The sequence SEQ ID NO: 7 corresponds to the following location: chr2:66438217-66438332. Its nucleic acid sequence is as follows:

AGCTGGCTAAAGGGCCCTTGTCCCCTCTCAGACTCCTTCAGCGGGCTGGAGTCCTCCC
TCGCTCGATTTCGCCCGAGAGCGTTAGGGGTTTCTAAATGCAGGCGCCTTTGTGTTGT
(SEQ ID NO: 7)

In a preferred embodiment, the sequence of the first polynucleotide biomarker consists of the nucleic acid sequence SEQ ID NO: 7.

In the table 1 below, it is disclosed:
- the nucleic acid sequence of the amplicon related to *MEIS1* (corresponding to the first polynucleotide biomarker having SEQ ID NO: 7),
- the nucleic acid sequence of the converted amplicon related to *MEIS1* wherein 100% of CpG sites were methylated (SEQ ID NO: 10),
- the nucleic acid sequence of the converted amplicon related to *MEIS1* wherein no CpG site was methylated (SEQ ID NO: 13),
- the nucleic acid sequence corresponding to the converted amplicon related to MEIS1 wherein the cytosine of the CpG sites are replaced with Y (Y being C or T) (SEQ ID NO: 16),
- the nucleic acid sequence corresponding to the amplicon related to *MEIS1* wherein all the cytosines are replaced with Y (Y being C or T) (SEQ ID NO: 19).

**Table 1**

| Name | SEQ ID NO | Nucleic acid sequence |
|---|---|---|
| Amplicon | 7 | AGCTGGCTAAAGGGCCCTTGTCCCCTCTCAGACTCCTTCAGC GGGCTGGAGTCCTCCCTCGCTCGATTTCGCCCGAGAGCGTTA GGGGTTTCTAAATGCAGGCGCCTTTGTGTTGT |
| Converted amplicon with 100% of CpG methylation | 10 | AGTTGGTTAAAGGGTTTTTGTTTTTTTTTAGATTTTTTTAGC GGGTTGGAGTTTTTTTTCGTTCGATTTCGTTCGAGAGCGTTA GGGGTTTTTAAATGTAGGCGTTTTTGTGTTGT |
| Converted amplicon with 0% of CpG methylation | 13 | AGTTGGTTAAAGGGTTTTTGTTTTTTTTTAGATTTTTTTAGT GGGTTGGAGTTTTTTTTTGTTTGATTTTGTTTGAGAGTGTTA GGGGTTTTTAAATGTAGGTGTTTTTGTGTTGT |
| Converted amplicon with C of CpG = Y | 16 | AGTTGGTTAAAGGGTTTTTGTTTTTTTTTAGATTTTTTTAGY GGGTTGGAGTTTTTTTTYGTTYGATTTYGTTYGAGAGYGTTA GGGGTTTTTAAATGTAGGYGTTTTTGTGTTGT |
| Amplicon (all C=>Y) | 19 | AGYTGGYTAAAGGGYYYTTGTYYYYTYTYAGAYTYYTTYAGY GGGYTGGAGTYYTYYYTYGYTYGATTTYGYYYGAGAGYGTTA GGGGTTTYTAAATGYAGGYGYYTTTGTGTTGT |

In some embodiments, the one or more CpG sites in the first polynucleotide biomarker is cg00995986. The locations of the CpG sites are given in the Illumina HumanMethylation450K Manifest.

An example of a pair of primers that amplifies the first polynucleotide biomarker having the nucleic acid sequence selected from the group consisting of SEQ ID NO: 7, SEQ ID NO: 10, SEQ ID NO: 13, SEQ ID NO: 16 and SEQ ID NO: 19 comprises a primer having the nucleic acid sequence AGTTGGTTAAAGGGTTTTTGTT (SEQ ID NO: 22) (forward primer) and a primer having the nucleic acid sequence ACAACACAAAAACRCCTACATTTAAAAAC (SEQ ID NO: 23) (reverse primer).

### Second biomarker

As shown in figure 3, the second polynucleotide biomarker is inside the gene *GDF6,* typically between exon 2 and exon 1.

In some embodiments, the second polynucleotide biomarker has a sequence comprised in the nucleic acid sequence SEQ ID NO: 2.

In some embodiments, the second polynucleotide biomarker has a sequence which is comprised in the nucleic acid sequence SEQ ID NO: 5.

In some embodiments, the sequence of second polynucleotide biomarker comprises the nucleic acid sequence SEQ ID NO: 8. The nucleic acid sequence SEQ ID NO: 8 corresponds to the following location: chr8:96159758-96159834. Its nucleic acid sequence is as follows:

GGCGCCAGGACCAGGGGGCTGCCCGACGCCGCTCGCGGACTAGTTCCTCAGACTGTGGGACTC
CCTAGTGCCGGCTT (SEQ ID NO: 8)

In a preferred embodiment, the sequence of the second polynucleotide biomarker consists of the nucleic acid sequence SEQ ID NO: 8.

In the table 2 below, it is set forth:
- the nucleic acid sequence of the amplicon related to *GDF6* (corresponding to the second polynucleotide biomarker having SEQ ID NO: 8),
- the nucleic acid sequence of the converted amplicon related to *GDF6* wherein 100% of CpG sites were methylated (SEQ ID NO: 11),
- the nucleic acid sequence of the converted amplicon related to *GDF6* wherein no CpG site was methylated (SEQ ID NO: 14),
- the nucleic acid sequence corresponding to the converted amplicon related to *GDF6* wherein the cytosine of the CpG sites are replaced with Y (Y being C or T) (SEQ ID NO: 17),
- the nucleic acid sequence corresponding to the amplicon related to *GDF6* wherein all the cytosines are replaced with Y (Y being C or T) (SEQ ID NO: 20).

**Table 2**

| Name | SEQ ID NO | Nucleic acid sequence |
|---|---|---|
| Amplicon | 8 | GGCGCCAGGACCAGGGGGCTGCCCGACGCCGCTCGCGGACTAGT TCCTCAGACTGTGGGACTCCCTAGTGCCGGCTT |
| Converted amplicon with 100% of CpG methylation | 11 | GGCGTTAGGATTAGGGGGTTGTTCGACGTCGTTCGCGGATTAGT TTTTTAGATTGTGGGATTTTTTAGTGTCGGTTT |
| Converted amplicon with 0% of CpG methylation | 14 | GGTGTTAGGATTAGGGGGTTGTTTGATGTTGTTTGTGGATTAGT TTTTTAGATTGTGGGATTTTTTAGTGTTGGTTT |
| Converted amplicon with C of CpG = Y | 17 | GGYGTTAGGATTAGGGGGTTGTTYGAYGTYGTTYGYGGATTAGT TTTTTAGATTGTGGGATTTTTTAGTGTYGGTTT |
| Amplicon (all C=>Y) | 20 | GGYGYYAGGAYYAGGGGYTGYYYGAYGYYGYTYGYGGAYTAGT TYYTYAGAYTGTGGGAYTYYYTAGTGYYGGYTT |

In some embodiments, the one or more CpG sites in the second polynucleotide biomarker is cg03224572. The locations of the CpG sites are given in the Illumina HumanMethylation450K Manifest.

An example of a pair of primers that amplifies the second polynucleotide biomarker having the nucleic acid sequence selected from the group consisting of SEQ ID NO: 8, SEQ ID NO: 11, SEQ ID NO: 14, SEQ ID NO: 17 and SEQ ID NO: 20 comprises a primer having the nucleic acid sequence GGYGTTAGGATTAGGGGGTTG (SEQ ID NO: 24) (forward primer) and a primer having the nucleic acid sequence AAACCRACACTAAAAAATCCCACAATCTA (SEQ ID NO: 25) (reverse primer).

### Third biomarker

As shown in figure 5, the third polynucleotide biomarker is inside the gene *MIR196A1,* typically in the CpG island located between 5'UTR of *HOXB7* and exon 2 of *HOX-AS4.*

In some embodiments, the third polynucleotide biomarker has a sequence comprised in the nucleic acid sequence SEQ ID NO: 3.

In some embodiments, the third polynucleotide biomarker has a sequence which is comprised in the nucleic acid sequence SEQ ID NO: 6.

In some embodiments, the sequence of the third polynucleotide biomarker comprises the nucleic acid sequence SEQ ID NO: 9. The nucleic acid sequence SEQ ID NO: 9 corresponds to the following location : chr17:48633954-48634042. Its nucleic acid sequence is as follows:

AGTCGGCGCTGACCCAGCCCGCGCCCGGCAGAGACTGGAAGCCGCCGCCTACACGGAAAATCCACAG
AGCCCCGCGCAGACCCTATGAT (SEQ ID NO: 9)

In a preferred embodiment, the sequence of the third polynucleotide biomarker consists of the nucleic acid sequence SEQ ID NO: 9.

In the table 3 below, it is disclosed:
- the nucleic acid sequence of the amplicon related to *MIR196A1* (corresponding to the third polynucleotide biomarker having SEQ ID NO: 9),
- the nucleic acid sequence of the converted amplicon related to *MIR196A1* wherein 100% of CpG sites were methylated (SEQ ID NO: 12),
- the nucleic acid sequence of the converted amplicon related to *MIR196A1* wherein no CpG site was methylated (SEQ ID NO: 15),
- the nucleic acid sequence corresponding to the converted amplicon related to *MIR196A1* wherein the cytosine of the CpG sites are replaced with Y (Y being C or T) (SEQ ID NO: 18),
- the nucleic acid sequence corresponding to the amplicon related to *MIR196A1* wherein all the cytosines are replaced with Y (Y being C or T) (SEQ ID NO: 21).

**Table 3**

| Name | SEQ ID NO | Nucleic acid sequence |
|---|---|---|
| Amplicon | 9 | AGTCGGCGCTGACCCAGCCCGCGCCCGGCAGAGACTGGAA GCCGCCGCCTACACGGAAAATCCACAGAGCCCCGCGCAGA CCCTATGAT |
| Converted amplicon with 100% of CpG methylation | 12 | AGTCGGCGTTGATTTAGTTCGCGTTCGGTAGAGATTGGAA GTCGTCGTTTATACGGAAAATTTATAGAGTTTCGCGTAGA TTTTATGAT |
| Converted amplicon with 0% of CpG methylation | 15 | AGTTGGTGTTGATTTAGTTTGTGTTTGGTAGAGATTGGAA GTTGTTGTTTATATGGAAAATTTATAGAGTTTTGTGTAGA TTTTATGAT |
| Converted amplicon with C of CpG = Y | 18 | AGTYGGYGTTGATTTAGTTYGYGTTYGGTAGAGATTGGAA GTYGTYGTTTATAYGGAAAATTTATAGAGTTTYGYGTAGA TTTTATGAT |
| Amplicon (all C=>Y) | 21 | AGTYGGYGYTGAYYYAGYYYGYGYYYGGYAGAGAYTGGAA GYYGYYGYYTAYAYGGAAAATYYAYAGAGYYYYGYGYAGA YYYTATGAT |

In some embodiments, the one or more CpG sites in the second polynucleotide biomarker is cg01452847. The locations of the CpG sites are given in the Illumina HumanMethylation450K Manifest.

An example of a pair of primers that amplifies the third polynucleotide biomarker having the nucleic acid sequence selected from the group consisting of SEQ ID NO: 9, SEQ ID NO: 12, SEQ ID NO: 15, SEQ ID NO: 18 and SEQ ID NO: 21 comprises a primer that hybridizes to the nucleic acid sequence AGTYGGYGTTGATTTAGT (SEQ ID NO: 26) (forward primer) and a primer that hybridizes to the nucleic acid sequence ATCATAAAATCTACRCRAAACTC (SEQ ID NO: 27) (reverse primer).

In an embodiment, the DNA methylation is detected and/or its level determined within 1, 2 or 3 of the at least one polynucleotide biomarkers as defined above, preferably within both the first, the second and the third polynucleotide biomarkers as defined above. Using the combination of the 3 biomarkers increases the sensitivity and the specificity of the test.

The present invention also relates to the use of the methods of detecting DNA methylation and/or of determining the level of DNA methylation as defined above for diagnosing and/monitoring a lung cancer, preferably NSCLC in a subject.

### B. Composition of primers and kit

In another aspect of the invention, it is provided a composition of primers as well as a kit for detecting and/or determining the level of DNA methylation at one or more CpG sites within at least one polynucleotide biomarker. The at least one polynucleotide biomarker and the CpG sites are as defined in section A above.

In some embodiments, the composition of primers as well as the kit are for detecting DNA methylation and/or determining the level of DNA methylation in at least 1, preferably 2 more preferably 3 of the polynucleotide biomarkers selected from the group consisting of the first polynucleotide biomarker, the second polynucleotide biomarker and the third polynucleotide biomarker.

In some embodiments, the composition of primers comprises at least one pair of primers able to produce the amplicon of the first, the second and/or the third polynucleotide biomarkers. Therefore, the composition of primers may comprise:
- a pair of primers able to produce the amplicon of the first polynucleotide biomarker having the nucleic acid sequence selected from the group consisting of SEQ ID NO: 7, SEQ ID NO: 16 and SEQ ID NO: 19, and/or
- a pair of primers able to produce the amplicon of the second polynucleotide biomarker having the nucleic acid sequence selected from the group consisting of SEQ ID NO: 8, SEQ ID NO: 17 and SEQ ID NO: 20,and/or
- a pair of primers able to produce the amplicon of the third polynucleotide biomarker having the nucleic acid sequence selected from the group consisting of SEQ ID NO: 9, SEQ ID NO: 18 and SEQ ID NO: 21.

The pair of primers able to produce the amplicon of the first polynucleotide biomarker may comprise a primer having the nucleic acid sequence SEQ ID NO: 22 and a primer having the nucleic acid sequence SEQ ID NO: 23.

The pair of primers able to produce the amplicon of the second polynucleotide biomarker may comprise a primer having the nucleic acid sequence SEQ ID NO: 24 and a primer having the nucleic acid sequence SEQ ID NO: 25,.

The pair of primers able to produce the amplicon of the third polynucleotide biomarker may comprise a primer having the nucleic acid sequence SEQ ID NO: 26 and a primer having the nucleic acid sequence SEQ ID NO: 27.

The present invention also relates to a kit for detecting and/or determining the level of DNA methylation comprising:
- means for detecting and/or determining the level of DNA methylation at one or more CpG sites within at least one polynucleotide biomarker, wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3,
and optionally DNA amplification reagents.

Reagents available for this purpose are well-known in the art. For example, they are commercialized by suppliers such as BIORAD (Biorad digital PCR with consumables such as Droplet generation oil for probes, ddPCR SuperMix for probes), QIAGEN or STILLA.

In some preferred embodiments of the kit of the invention, the primers, and optional reagents are in lyophilised form to allow ambient storage.

The components of the kits are packaged together into any of the various containers suitable for nucleic acid amplification such as plates, slides, wells, dishes, beads, particles, cups, strands, chips, strips and others.

The kit optionally includes instructions for performing at least one specific embodiment of the method of the invention. In some advantageous embodiments, the kit comprises micro-well plates or microtubes, preferably in a dried format, i.e., wherein the wells of the plates or microtubes comprise a dried composition containing at least the primers.

Another aspect of the invention is the use of the composition of primers of the invention, or of the kit of the invention for diagnosing and/monitoring a lung cancer, preferably NSCLC in a subject.

The present invention also relates to the use of an agent for detecting and/or determining the level of DNA methylation at one or more CpG sites within at least one polynucleotide biomarker selected from the group consisting of a first polynucleotide biomarker, a second polynucleotide biomarker and a third polynucleotide biomarker as disclosed at section A in the manufacture of a reagent for a method for diagnosing a lung cancer in a subject according to the invention and/or a method of monitoring according to the invention.

### C. Method of diagnosing

In an aspect of the invention, the present invention relates to a method of diagnosing lung cancer in a subject, the method of diagnosing comprising the step of detecting DNA methylation at one or more CpG sites within at least one polynucleotide biomarker in a biological sample obtained from said subject,
wherein the presence of detected DNA methylation is indicative that the subject has a lung cancer. The step of detecting DNA methylation, the at least one biomarker and the one or more CpG sites are as disclosed at section A above.

"Diagnosis" as used herein generally includes determination as to whether a subject is likely affected by a given disease, disorder or dysfunction.

In some embodiments, the present invention relates to a method of diagnosing lung cancer in a subject, the method of diagnosing comprising the step of detecting DNA methylation at one or more CpG sites within at least one polynucleotide biomarker in a biological sample obtained from the subject,
wherein said at least one polynucleotide biomarker is selected from the group consisting of:
- a first polynucleotide biomarker having a sequence comprised in the nucleic acid sequence SEQ ID NO: 1, preferably comprised in the nucleic acid sequence SEQ ID NO: 4, more preferably which comprises or consists of the nucleic acid sequence SEQ ID NO: 7,
- a second polynucleotide biomarker having a sequence comprised in the nucleic acid sequence SEQ ID NO: 2, preferably comprised in the nucleic acid sequence SEQ ID NO: 5, more preferably which comprises or consists of the nucleic acid sequence SEQ ID NO: 8,
   and
- a third polynucleotide biomarker having a sequence comprised in the nucleic acid sequence SEQ ID NO: 3, preferably comprised in the nucleic acid sequence SEQ ID NO: 6, , more preferably which comprises or consists of the nucleic acid sequence SEQ ID NO: 9,
wherein the presence of detected methylated genomic DNA is indicative that the subject has a lung cancer.

In an embodiment, the absence of detected DNA methylation at one or more CpG sites is indicative that the subject hasn't a lung cancer.

Preferably, the lung cancer is a non-small cell lung cancer (NSCLC).

In one embodiment, the subject is suspected of having, or has had a lung cancer, preferably a non-small cell lung cancer (NSCLC).

In a preferred embodiments, the method of diagnosis is *in vitro.*

Advantageously, the biological sample is a liquid biological sample. Preferably, the biological sample is selected from the group consisting of blood, blood derived sample such as serum or plasma, cerebrospinal fluid, urine, sweat, saliva, tracheal washing, bronchial washing, pleural liquid, ascites and cerebrospinal fluid, more preferably the biological sample is blood or a blood derived sample.

The use of liquid biological samples presents many advantages notably compared to tissue sample. It is non-invasive, quick and easy. It is repeatable and allows dynamic monitoring. Moreover, there is a diversity of possible liquid biological samples.

Thus, the polynucleotide biomarker(s) according to the invention have advantageously been optimized for liquid biopsy.

In an embodiment, the DNA methylation is detected within 1, 2 or 3 of the at least one polynucleotide biomarkers as defined above, preferably within both the first, the second and the third polynucleotide biomarkers as defined above.

The present invention also relates to an in *vitro* or *ex vivo* method for obtaining data to aid in the diagnosis of lung cancer comprising the step of detecting DNA methylation as defined in section A above.

### C. Method for monitoring

In an aspect of the invention, the present invention relates to a method of monitoring lung cancer in a subject, the method of monitoring comprising the step of determining the level DNA methylation as defined at section A above in a biological sample obtained from the subject, during a treatment procedure or during a certain period of time, for example during at least 1 week, 2 weeks, 3 weeks, 4 weeks, 1 month, 2 months, 3 months, 4 months, 5 months, 6 months, 1 year, 2 years, 3 years, 5 years, 10 years, or any other period of time. Therefore, in some embodiments, the method of monitoring a lung cancer in a subject, comprises the steps of :
(a) determining the level of DNA methylation at one or more CpG sites within at least one polynucleotide biomarker in a first biological sample obtained from the subject at a first time point,
   wherein said at least one polynucleotide biomarker is selected from the group consisting of:
   - a first polynucleotide biomarker having a sequence comprised in the nucleic acid sequence SEQ ID NO: 1, preferably comprised in the nucleic acid sequence SEQ ID NO: 4, more preferably which comprises or consists of the nucleic acid sequence SEQ ID NO: 7,
   - a second polynucleotide biomarker having a sequence comprised in the nucleic acid sequence SEQ ID NO: 2, preferably comprised in the nucleic acid sequence SEQ ID NO: 5, more preferably which comprises or consists of the nucleic acid sequence SEQ ID NO: 8,
      and
   - a third polynucleotide biomarker having a sequence comprised in the nucleic acid sequence SEQ ID NO: 3, preferably comprised in the nucleic acid sequence SEQ ID NO: 6, , more preferably which comprises or consists of the nucleic acid sequence SEQ ID NO: 9,
(b) determining the level of DNA methylation at one or more CpG sites in said at least one polynucleotide biomarker in a second biological sample obtained from the subject later at a second time point,
   and
(c) comparing the level of DNA methylation determined in the second biological sample with the level of DNA methylation determined in the first biological sample,
   - wherein an increase between the level of DNA methylation determined in the second biological sample and the level of DNA methylation determined in the first sample is indicative that the lung cancer is progressing,
   - wherein a decrease between the level of DNA methylation determined in the second biological sample and the level of DNA methylation determined in the first sample is indicative that the lung cancer is not progressing.

Whether an increase or a decrease in DNA methylation is statistically significant can be determined easily by the person skilled in the art using several well-known statistical evaluation tools, for example, determination of confidence intervals, determination of p values, Student's t-test, Mann-Whitney test, etc.

For example, the determined level of DNA methylation in a sample at a first time point may corresponds to the number of copies with a given DNA methylation at one or more CpG in said sample. If there is an increase in said determined level of DNA methylation (i.e. the number of copies with a given DNA methylation at one or more CpG) in a sample obtained from the same subject later at a second time point, it is indicative that is indicative that the lung cancer is progressing.

In some embodiments, the method of monitoring is a method of monitoring the efficiency of a treatment in a subject suffering from a lung cancer. The method of monitoring the efficiency of a treatment may comprise:
(a) determining the level of DNA methylation at one or more CpG sites within at least one polynucleotide biomarker, in a biological sample obtained from the subject before or at the beginning of the treatment,
   wherein the polynucleotide biomarker is selected from the group consisting of:
   - a first polynucleotide biomarker having a sequence comprised in the nucleic acid sequence SEQ ID NO: 1, preferably comprised in the nucleic acid sequence SEQ ID NO: 4, more preferably which comprises or consists of the nucleic acid sequence SEQ ID NO: 7,
   - a second polynucleotide biomarker having a sequence comprised in the nucleic acid sequence SEQ ID NO: 2, preferably comprised in the nucleic acid sequence SEQ ID NO: 5, more preferably which comprises or consists of the nucleic acid sequence SEQ ID NO: 8,
      and
   - a third polynucleotide biomarker having a sequence comprised in the nucleic acid sequence SEQ ID NO: 3, preferably comprised in the nucleic acid sequence SEQ ID NO: 6, more preferably which comprises or consists of the nucleic acid sequence SEQ ID NO: 9,
(b) determining the level of DNA methylation at one or more CpG sites in said at least one polynucleotide biomarker, in a biological sample obtained from the subject during or after the treatment, and
(c) comparing the DNA methylation detected at one or more CpG sites before or at the beginning of the treatment with the DNA methylation detected at one or more CpG sites during or after the treatment,
   - wherein an increase or an equality between the level of DNA methylation determined before or at the beginning of and the level of DNA methylation determined during or after the treatment is indicative that the treatment is not efficient,
   - wherein a decrease between the level of DNA methylation determined before or at the beginning of and the level of DNA methylation determined during or after the treatment is indicative that the treatment is efficient.

Preferably, the lung cancer is a non-small cell lung cancer (NSCLC).

The treatment may for example comprises a surgery, a chemotherapy, an immunotherapy or a radiation therapy.

In a preferred embodiment, the method of monitoring is an *in vitro* method.

As for the method of diagnosing, the biological sample is preferably a liquid biological sample.

In an embodiment, the level DNA methylation is determined within 1, 2 or 3 of the at least one polynucleotide biomarkers, preferably within both the first, the second and the third polynucleotide biomarkers.

The present invention also relates to an in *vitro* or ex *vivo* method for obtaining data to aid in the monitoring of lung cancer comprising the step of determining the level of DNA methylation as disclosed in section A above.

### D. Method for treating

In an aspect of the invention, it is provided a method of treatment comprising the method of detecting the DNA methylation of the invention, the use of the composition of primers of the invention, or of the kit of the invention or the method of diagnosing according to the invention and a step of treating lung cancer of a subject for which the DNA methylation is detected in its biological sample.

Therefore, in some embodiments, the present invention relates to a method for treating lung cancer in a subject, the method of treating comprising the steps of:
(a) detecting DNA methylation at one or more CpG sites within at least one polynucleotide biomarker, in a biological sample obtained from a subject,
   wherein said at least one polynucleotide biomarker is selected from the group consisting of:
   - a first polynucleotide biomarker
   - a second polynucleotide biomarker, and
   - a third polynucleotide biomarker
(b) treating the subject for the lung cancer, if the presence of methylated genomic DNA is detected ; said at least one polynucleotide biomarker being as disclosed at section A.

It is also provided a method of treatment comprising the method of determining the level of DNA methylation of the invention, the use of the composition of primers of the invention, or of the kit of the invention or the method of monitoring according to the invention and a step of treating lung cancer of a subject. The subject may be for example a subject wherein an increase or no decrease in DNA methylation has been determined in its biological sample. Therefore, in some embodiments, the present invention relates to a method for treating lung cancer in a subject, the method of treating comprising the steps of:
- determining the level of DNA methylation at one or more CpG sites within at least one polynucleotide biomarker in a first biological sample obtained from the subject at a first time point and in a second biological sample obtained from the subject at a second time point determining the level DNA methylation, wherein said at least one polynucleotide biomarker is selected from the group consisting of the first polynucleotide biomarker, the second polynucleotide biomarker, and the third polynucleotide biomarker
- treating the subject for the lung cancer, if an increase between the level of DNA methylation in the second biological sample and in the first sample is determined; the at least one polynucleotide biomarker being as disclosed at section A.

In particular, said at least one polynucleotide biomarker may be selected from the group consisting of:
- a first polynucleotide biomarker having a sequence comprised in the nucleic acid sequence SEQ ID NO: 1, preferably comprised in the nucleic acid sequence SEQ ID NO: 4, more preferably which comprises or consists of the nucleic acid sequence SEQ ID NO: 7,
- a second polynucleotide biomarker having a sequence comprised in the nucleic acid sequence SEQ ID NO: 2, preferably comprised in the nucleic acid sequence SEQ ID NO: 5, more preferably which comprises or consists of the nucleic acid sequence SEQ ID NO: 8,
   and
- a third polynucleotide biomarker having a sequence comprised in the nucleic acid sequence SEQ ID NO: 3, preferably comprised in the nucleic acid sequence SEQ ID NO: 6, , more preferably which comprises or consists of the nucleic acid sequence SEQ ID NO: 9.

In an embodiment, the level DNA methylation is determined within 1, 2 or 3 of the at least one polynucleotide biomarkers, preferably within both the first, the second and the third polynucleotide biomarkers.

Preferably, the lung cancer is a non-small cell lung cancer (NSCLC).

The treatment may for example comprises a surgery, a chemotherapy, an immunotherapy or a radiation therapy.

### EXAMPLES

### Material and Methods:

### DNA isolation and bisulfite modification

Tumors DNA was extracted from frozen biopsies and Formalin-Fixed Paraffin-Embedded (FFPE) samples. In EDTA collected blood samples were pre-treated to obtain supernatants which were stored at -80°C. Circulating cell-free DNA (cfDNA) was extracted from 4 mL to 6 mL of plasma using the QIAamp^{®} Circulating Nucleic Acid kit (Qiagen^{®}, Hilden, Germany) according to the manufacturer's protocol and resuspended in 50 µL of buffer. The quantity of DNA was measured by Qubit 2.0 fluorometer (Invitrogen^{®}, Life Technologies).

For all samples, bisulfite treatment was performed to convert unmethylated cytosine into thymine without changing methylated cytosine, by using the EpiTect Fast Bisulfite Kit^{®} (QIAGEN) from 20 µL of previously extracted DNA (except for DNA concentration above 10 ng/µL where 10µL was used or below depending of the DNA concentration value).

### Development of the digital PCR analysis

It was developed an assay targeting these biomarkers previously adapted on a Naica Crystal Digital PCR system^{™} (Stilla Technonologies, Villejuif, France). After bisulfite conversion, a volume of 1-10 µL of DNA extract was assembled in a 25 µL PCR mixtures containing 5 µL of Naica Multiplex PCR Buffer A 5X (1X final concentration), 1 µL of 1.25% diluted (0.05% final concentration) Bovine Serum Albumin (Sigma-Aldrich) and 1,3 µL of a 20X concentrated mix of primers and hydrolysis probes. The probe and primer sequences were designed and described in Table 4.

**Table 4**

| Biomarker | Forward primer (Y = C or T) | Reverse primer (R= A or G) | Hydrolysis probe |
|---|---|---|---|
| 1st | AGT-TGG-TTA-AAG- GGT-TTT-TGT-T (SEQ ID NO: 22) | ACA-ACA-CAA-AAA- CRC-CTA-CAT-TTA- AAA-AC (SEQ ID NO: 23) | CGTTCGAGAGCGTTAGGG (SEQ ID NO: 28) |
| 2nd | GGY-GTT-AGG-ATT- AGG-GGG-TTG (SEQ ID NO: 25) | AAA-CCR-ACA-CTA- AAA-AAT-CCC-ACA- ATC-TA (SEQ ID NO: 26) | TTCGACGTCGTTCGCG (SEQ ID NO: 29) |
| 3rd | AGT-YGG-YGT-TGA- TTT-AGT (SEQ ID NO: 27) | ATC-ATA-AAA-TCT- ACR-CRA-AAC-TC (SEQ ID NO: 28) | TCGCGTTCGGTAGAGATTG (SEQ ID NO: 30) |

PCR mixtures were carefully loaded into wells of a Sapphire chip (Stilla Technologies). Then the PCR amplification was performed on the Naica Geode as followed: droplet generation and partitioning step, then 95°C for 3 minutes, followed by 45 cycles of 94°C for 45 seconds, 62°C for 30 seconds and 65°C for 30 seconds and then a final pressure release step.

After PCR amplification, Sapphire chips were transferred into the Naica Prism3 scanner and fluorescence imaging was done using the following exposure times: blue channel= 70 ms, green channel: 250 ms, red channel= 55 ms. Digital PCR results were analyzed using CrystralMiner software (version 4.0.10.3). Polygon thresholding mode was set in order distinguish *GDF6* and *MIR196A1* positive droplets (positive signal in the same channel, red). Finally, data where exported in excel files (Microsoft) in order to calculate fractions of methylated alleles for each marker.

## Claims

1. A method of diagnosing lung cancer, preferably a non-small cell lung cancer (NSCLC), in a subject, the method of diagnosing comprising the step of detecting DNA methylation at one or more CpG sites within at least one polynucleotide biomarker, in a biological sample obtained from said subject,
wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3 .
wherein the presence of detected DNA methylation at one or more CpG sites is indicative that the subject has a lung cancer.

2. A method of monitoring a lung cancer, preferably a NSCLC, in a subject, the method of monitoring comprising the steps of:
(a) determining the level of DNA methylation at one or more CpG sites within at least one polynucleotide biomarker, in a first biological sample obtained from said subject at a first time point and
wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3,
(b) determining the level of DNA methylation at one or more CpG sites within said at least one polynucleotide biomarker in a second biological sample obtained from said subject later at a second time point, and
(c) comparing the level of DNA methylation determined in the second biological sample with the level of DNA methylation determined in the first biological sample,
- wherein an increase between the level of DNA methylation determined in the second biological sample and the level of DNA methylation determined in the first biological sample is indicative that the lung cancer is progressing,
- wherein a decrease between the level of DNA methylation determined in the second biological sample and the level of DNA methylation determined in the first biological sample is indicative that the lung cancer is not progressing.

3. A method of monitoring the efficiency of a treatment in a subject suffering from a lung cancer, preferably a NSCLC, the method of monitoring comprising the steps of:
(a) determining the level of DNA methylation at one or more CpG sites within at least one polynucleotide biomarker, in a biological sample obtained from said subject before or at the beginning of the treatment,
wherein said at least one polynucleotide biomarker is selected from the group consisting of:
- a first polynucleotide biomarker having a sequence comprised in the sequence SEQ ID NO: 1,
- a second polynucleotide biomarker having a sequence comprised in the sequence SEQ ID NO: 2, and
- a third polynucleotide biomarker having a sequence comprised in the sequence SEQ ID NO: 3,
(b) determining the level of DNA methylation at one or more CpG sites in said at least one polynucleotide biomarker, in a biological sample obtained from said subject during or after the treatment, and
(c) comparing the level of DNA methylation determined before or at the beginning of the treatment with the level of DNA methylation determined during or after the treatment,
- wherein an increase or an equality between the level of DNA methylation determined before or at the beginning of and during or after the treatment is indicative that the treatment is not efficient,
- wherein a decrease between the level of DNA methylation determined before or at the beginning of and during or after the treatment is indicative that the treatment is efficient.

4. A method for treating lung cancer in a subject, the method of treating comprising the steps of:
(a) detecting DNA methylation at one or more CpG sites within at least one polynucleotide biomarker in a biological sample obtained from said subject,
wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3,
(b) treating the subject for the lung cancer, if the presence of DNA methylation at one or more CpG sites is detected.

5. The method according to any one of claims 1 to 4, wherein the DNA methylation is detected and/or the level of DNA methylation determined within at least two of the polynucleotide biomarkers, preferably within the three polynucleotide biomarkers.

6. A kit for detecting DNA methylation comprising:
- means for detecting and/or determining the level of DNA methylation at one or more CpG sites within at least one polynucleotide biomarker, wherein said at least one polynucleotide biomarker is selected from the group consisting of:
- a first polynucleotide biomarker having a sequence comprised in the nucleic acid sequence SEQ ID NO: 1,
- a second polynucleotide biomarker having a sequence comprised in the nucleic acid sequence SEQ ID NO: 2, and
- a third polynucleotide biomarker having a sequence comprised in the nucleic acid sequence SEQ ID NO: 3,
and optionally reagents for DNA amplification.

7. The method according to any one of claims 1 to 5 or the kit according to claim 6, wherein :
- the first polynucleotide biomarker has a sequence which is comprised in the nucleic acid sequence SEQ ID NO: 4, and/or
- the second polynucleotide biomarker has a sequence is comprised in the nucleic acid sequence SEQ ID NO: 5, and/or
- the third polynucleotide biomarker has a sequence is comprised in the nucleic acid sequence SEQ ID NO: 6.

8. The method or the kit according to any preceding claims, wherein:
- the first polynucleotide biomarker comprises the nucleic acid sequence SEQ ID NO: 7, and/or
- the second polynucleotide biomarker comprises the nucleic acid sequence SEQ ID NO: 8, and/or
- the third polynucleotide biomarker comprises the nucleic acid sequence SEQ ID NO: 9.

9. The method or the kit according to any preceding claims, wherein:
- the first polynucleotide biomarker consists of the nucleic acid sequence SEQ ID NO: 7, and/or
- the second polynucleotide biomarker consists of the nucleic acid sequence SEQ ID NO: 8, and/or
- the third polynucleotide biomarker consists of the nucleic acid sequence SEQ ID NO: 9.

10. The method or the kit according to any preceding claims wherein:
- the one or more CpG sites in the first polynucleotide biomarker is cg00995986; :
- the one or more CpG sites in the second polynucleotide biomarker is cg03224572 ;
- the one or more CpG sites in the third polynucleotide biomarker is cg01452847.

11. The method or the kit according to any preceding claims, wherein the biological sample is a liquid biological sample.

12. The method or the kit according to any preceding claims, wherein the at least one polynucleotide biomarker is from genomic DNA, more preferably from cell-free DNA (cfDNA).

13. Use of the kit according to any one of claims 6 to 12 for diagnosing and/or monitoring a lung cancer, preferably NSCLC in a subject.
